# FASCICULE DE BREVET EUROPEEN

(11) **EP 0 781 341 B1**
(45) Date de publication et mention de la délivrance du brevet: **20.08.2008**
(21) Numéro de dépôt: 95931242.2
(22) Date de dépôt: 14.09.1995
(51) Int. Cl.: C12N 15/68, C12N 15/70, C12N 1/21, C07K 14/50

(54) **PROCEDE DE PRODUCTION DE PROTEINES RECOMBINANTES, PLASMIDES ET CELLULES MODIFIEES**
HERSTELLUNGSVERFAHREN FÜR REKOMBINANTE PROTEINE, PLASMIDE UND MODIFIZIERTE ZELLEN
METHOD FOR THE PRODUCTION OF RECOMBINANT PROTEINS, PLASMIDS AND MODIFIED CELLS

(30) Priorité: 16.09.1994 FR 9411049
(43) Date de publication de la demande: 02.07.1997
(73) Titulaire: Aventis Pharma S.A., 92160 Antony (FR)
(72) Inventeur: CAMERON, Béatrice, F-75005 Paris (FR); CROUZET, Joel, F-92330 Sceaux (FR)
(74) Mandataire: Le Coupanec, Pascale A.M.P.
(86) Numéro de dépôt international: PCT/FR1995/001178
(87) Numéro de publication internationale: WO 1996/008572

(56) Documents cités:
- EP-A- 0 115 613
- EP-A- 0 360 006
- EP-A- 0 406 738
- WO-A-84/01172
- WO-A-91/16439
- FR-A- 2 642 086
- J. BIOTECHNOLOGY, vol. 28, no. 2,3, ELSEVIER, AMSTERDAM,NL, pages 291-299, C. HAIGERMOSER ET AL. 'Stability od r-microbes: Stabilization of plasmid vectors by the patitioning function of broad-host-range plasmid RP4'
- PROC. NATL.ACAD SCI., vol. 82, no. 4, NATL. ACAD SCI.,WASHINGTON,DC,US;, pages 1074-1078, S. TABOR AND C.C. RICHARDSON 'A bacteriophage T7 RNA polymerase/promoter system for controlled exclusive expression od specific genes'
- METHODS IN ENZYMOLOGY, vol. 185, ACADEMIC PRESS, INC., NEW YORK, US, pages 60-89, F.W. STUDIER ET AL. 'Use of T7 RNA polymerase to direct expression of cloned genes' cité dans la demande
- GENE (AMST) 110 (1). 1992. 105-108. CODEN: GENED6 ISSN: 0378-1119, CROUZET J ET AL 'CONSTRUCTION OF A BROAD-HOST-RANGE NON-MOBILIZABLE STABLE VECTOR CARRYING RP4 PAR-REGION.'

## Description

La présente invention concerne la production de protéines recombinantes chez les bactéries. Elle concerne plus particulièrement un nouveau procédé permettant une production élevée de protéines recombinantes chez les bactéries, mettant en oeuvre un système d'expression particulièrement stable et efficace comprenant un promoteur du bactériophage T7 et une région de stabilisation. La présente invention concerne également les plasmides utilisables dans ce procédé, et les protéines recombinantes ainsi produites.

Les bactéries, et en particulier E. coli, peuvent être utilisées pour produire des protéines de diverses origines, aussi bien de procaryotes que d'eucaryotes. En particulier, des protéines humaines peuvent être produites chez E. coli. Pour ce faire le gène de structure, ou le cDNA du gène codant pour la protéine en question doit être placé derrière des signaux d'expression appropriés (promoteur et site de fixation des ribosomes) reconnus par la machinerie d'expression d'E. coli. Le système le plus efficace utilisé chez E. coli est le système décrit par Studier (Studier et al., 1990). Ce système consiste en l'utilisation d'un promoteur du phage T7 qui est reconnu spécifiquement par l'ARN polymérase de ce même bactériophage (E. coli ne possédant per se aucun promoteur reconnu par cette même polymérase). Dans ce système d'expression, il y a une induction, par exemple à l'IPTG, de l'expression du gène de l'ARN polymérase du phage T7 (celui-ci étant positionné sous contrôle d'un promoteur inductible tel que PlacUV5 d'E. coli ). De cette induction résulte l'expression du gène placé sous contrôle du promoteur du bactériophage T7 reconnu par la polymérase. Comme la polymérase ne reconnaît que le promoteur en amont du gène à exprimer, celui-ci est exprimé, en général, à un niveau très élevé (au dessus de quelques pour-cent des protéines totales bactériennes). De très nombreuses publications font état aujourd'hui de l'utilisation de ce système initialement décrit par Studier (Studier et al., 1990).

Ainsi la demande EPO 406 738 décrit un vecteur d'expression pour produire le aFGF comprenant le promotteur T7.

Comme indiqué ci-dessus, les bactéries, et en particulier E. coli, sont utilisées pour la production de protéines humaines d'intérêt pharmaceutique. Si ces dites protéines doivent être ensuite utilisées dans diverses thérapies, il convient de les préparer en respectant les règles de Bonne Pratique de Production (BPL). Pour ce faire il est indispensable de posséder un système de production qui soit reproductible et qui assure l'obtention d'un produit de qualité hautement définie ayant une très grande pureté. Un des aspects important d'un procédé de production d'une protéine d'intérêt pharmaceutique réside dans la souche qui permet la production de la dite protéine. En effet il faut que cette souche assure un mode de production reproductible tant au niveau qualitatif que quantitatif.

Au niveau qualitatif, par exemple, il convient de produire une protéine d'une homogénéité totale, c'est à dire ayant une séquence primaire identique à celle de la protéine naturelle. En effet, si dans une cellule bactérienne, au cours de la fermentation, il se produisait une mutation ponctuelle qui modifie la séquence codée de la protéine à exprimer, il en résulterait la production d'une protéine modifiée mélangée avec la protéine de séquence sauvage. Une fois chez l'homme, cette protéine modifiée pourrait entraîner une réaction immunitaire qui pourrait être très néfaste au traitement, ou bien, lors d'un traitement ultérieur, entraîner cette fois-ci une réaction immunitaire sévère.

Au niveau quantitatif, il est admis qu'un procédé doit être reproductible. Cela constitue un gage de la qualité du produit obtenu. Ainsi, il faut qu'il y ait entre les différents lots de production le même taux de synthèse de la protéine en question par unité de biomasse, dans la même unité de temps en utilisant les mêmes conditions de culture et de croissance. Un aspect très important de cette reproductibilité du niveau de la production de la protéine réside dans la présence du plasmide portant la cassette d'expression dans les cellules bactériennes. Un procédé de fermentation est d'autant mieux maîtrisé que toutes les cellules ont le plasmide en fin de culture (avant et après la production de la protéine). Pour maintenir le plasmide dans les bactéries on ajoute en général dans le milieu de culture un antibiotique sélectif pour la présence du plasmide. Cela peut poser des problèmes divers: coût de l'antibiotique au niveau de la préparation du milieu du fermenteur; comme l'antibiotique ne peut pas être stérilisé par autoclavage, il convient de l'ajouter extemporanément au milieu de culture; l'antibiotique peut ne pas être stable et générer des produits de transformation toxiques chez l'homme, ou du moins conduisant chez certains patients à des effets secondaires indésirables; l'antibiotique peut aussi, par lui-même être toxique chez l'homme, ou du moins conduire, chez certains patients, à des effets secondaires indésirables. Dans les deux derniers cas, il faudra montrer que dans le produit purifié, les traces de produits toxiques correspondant soit à l'antibiotique, soit à des produits dérivés sont inférieures, par doses administrables, à des niveaux susceptible, de procurer des effets secondaires ou toxiques. Ceci se traduit par des études lourdes et coûteuses.

La présente invention permet de remédier à ces problèmes. La présente invention fournit en effet un système d'expression particulièrement efficace, dans lequel l'antibiotique n'est pas nécessaire pour le maintien du plasmide au cours des générations bactériennes se déroulant dans le fermenteur. La présente invention réside en particulier dans la construction d'un système d'expression plasmidique dans lequel l'expression se fait grâce à un promoteur du bactériophage T7, et comportant en plus une région stabilisatrice. Le système selon l'invention est particulièrement avantageux puisque, contrairement à ce qui est observé pour le système de Studier et al., il permet de maintenir les plasmides dans toutes les cellules bactériennes après l'induction de l'expression de la protéine à exprimer, dans des conditions de culture où il n'y a pas d'antibiotique, c'est à dire sans pression de sélection. Les plasmides de Studier (Studier et al., 1990), après induction de l'expression, ne sont retrouvés que dans une fraction très faible des cellules. Il est clair que le système de l'invention stabilise fortement les plasmides, ce qui augmente les niveaux de production de protéines recombinantes et la reproductibilité du procédé. La région stabilisatrice utilisée pour la construction des plasmides selon l'invention est plus précisément dérivée du fragment par du plasmide RP4.

Un premier objet de l'invention réside donc dans un plasmide d'expression comprenant une séquence d'acide nucléique d'intérêt sous le contrôle d'un promoteur du bactériophage T7 et une région stabilisatrice comprenant tout ou partie de la région par du plasmide RP4 ou d'un dérivé de celle-ci. et comportant en outre l'opérateur lacO et le gène lacI^{q}.

Préférentiellement, le promoteur du bactériophage T7 utilisé dans les plasmides de la présente invention est le promoteur du gène 10.

Comme indiqué ci-avant, la région de stabilisation utilisée dans les plasmides de l'invention est dérivée du fragment par du plasmide RP4 (Gerlitz et al., 1990). Ce fragment porte différentes fonctions (en particulier 5 gènes désignés parA, parB, parC, parD et parE), et notamment une protéine devant avoir une activité recombinase site spécifique permettant probablement de résoudre les dimères plasmidiques (Eberl et al., 1994). Ce fragment par peut être isolé à partir du plasmide RP4 et cloné sur les plasmides d'expression de la présente invention, comme décrit dans les exemples. En outre, ce fragment peut être modifié avant ou après son introduction dans les plasmides de l'invention. Ainsi, la région stabilisatrice des plasmides de l'invention peut être constituée par tout ou partie de la région par du plasmide RP4 ou d'un dérivé de celle-ci.

En particulier, la région stabilisatrice des plasmides peut être constituée de l'insert PstI du plasmide pTUG3 ou des sous-fragments SphI-SphI-SphI du pOH23 ou SphI-SphI-ClaI du pOH41 ou SphI-SphI-BamHI du pGMA28 ou du SphI-SphI-BamHI du pGMA27qui sont décrits par Gerlitz et al., 1990 ou de tout sous-fragment de l'insert PstI du pTUG3 contenant au minimum l'insert SphI-SphI-BamHI du pGMA27. Alors que l'insert PstI du pTUG3 contient les gènes parCBA-parDE et les régions flanquantes, les inserts du pGMA27 ou pGMA28 ne contiennent que les gènes parCBA-parDE' et la région 5'flanquante. La région stabilisatrice peut également être constituée des gènes parDE ou du gène parD et de sa région promotrice ou d'un autre promoteur comme cela a déjà été décrit par Roberts et al, 1994. Cette région peut être obtenue à partir du fragment StyI-ClaI contenu dans le plasmide pTUG3. La région stabilisatrice peut aussi être constituée de toute combinaison de deux, trois ou quatre gènes par contenus dans le plasmide pTUG3, par exemple parA-parD ou patB-parB ou parAC-parD ou parBA-parDE avec leur région promotrice ou un autre promoteur.

Le terme dérivé de la région par comprend tout fragment obtenu par modification génétique et/ou chimique de la région par, et apte à la réalisation de plasmides stables selon l'invention. En particulier, ces dérivés peuvent être obtenus par délétions, mutations, additions, clivages, ligations, etc au niveau de la région par du plasmide RP4, selon les techniques connues de l'homme du métier. Ensuite, la fonctionnalité des dérivés peut être testée comme décrit dans les exemples (voir exemple 2. notamment).

Avantageusement, dans les plasmides selon la présente invention, la région stabilisatrice comprend une partie de la région par du plasmide RP4. Dans un mode particulier de réalisation, la région stabilisatrice est constituée du fragment compris entre les résidus 6038 et 8499 de la séquence SEQ ID n° 1.

Les plasmides de l'invention comportent en outre l'opérateur lacO et le gène lacI^{q}. Il a en effet été constaté que malgré la présence d'un promoteur du bactériophage T7 spécifique d'une polymérase non présente chez E. coli, une expression basale de la séquence d'acide nucléique d'intérêt se produit sans inducteur. Cette expression résiduelle peut induire une certaine baisse de stabilité du plasmide. La présence, dans les plasmides de l'invention, de l'opérateur lacO et du gène lacI^{q} permet avantageusement d'obtenir une expression plus régulée de la séquence d'acide nucléique et en particulier, d'inhiber toute expression en l'absence d'inducteur. Les plasmides incorporant ces éléments ont donc une stabilité et un contrôle de l'expression accrus. Pour obtenir l'effet recherché, l'opérateur lacO est avantageusement placé en aval du promoteur du bactériophage T7 et en amont de la séquence d'acide nucléique d'intérêt comme indiqué dans les exemples. Le gène lacI^{q} est inséré dans le plasmide, préférentiellement avec son propre promoteur, dans une région non essentielle pour les propriétés recherchées du plasmide. Ainsi, le gène est préférentiellement inséré en dehors de la région par et de la cassette d'expression de la séquence d'acide nucléique d'intérêt. Les séquences de cet opérateur et du gène lacI^{q} sont données dans la séquence SEQ ID n° 1. Il est entendu que ces éléments peuvent être remplacés par des séquences homologues ou possédant des fonctions équivalentes.

Dans un mode particulièrement préféré de mise en oeuvre, les plasmides selon l'invention comportent également un terminateur de transcription, situé en aval de la séquence d'acide nucléique d'intérêt. Avantageusement, le terminateur de transcription utilisé est celui du gène du bactériophage T7 dont le promoteur est utilisé. Préférentiellement, il s'agit du terminateur TΦ du bactériophage T7.

La séquence d'acide nucléique d'intérêt présente dans les plasmides selon l'invention peut être toute séquence codant pour une protéine d'intérêt pharmaceutique, agroalimentaire, ou pouvant être utilisée pour de la biocatalyse. Il peut s'agir d'un gène de structure, d'une séquence d'ADN complémentaire, d'une séquence synthétique, semi-synthétique, etc.

Préférentiellement, la séquence d'acide nucléique code pour une protéine d'intérêt pharmaceutique choisie par exemple parmi les enzymes, les dérivés sanguins, les hormones, les lymphokines (interleukines, interférons, TNF, etc), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc; les apolipoprotéines : ApoAI, ApoAIV, ApoE, etc, la dystrophine ou une minidystrophine, la protéine CFTR associée à la mucoviscidose, les gènes suppresseurs de tumeurs : p53, Rb, Rap1A, DCC, k-rev, etc, les gènes codant pour des facteurs impliqués dans la coagulation : Facteurs VII, VIII, IX, les gènes intervenant dans la réparation de l'ADN, etc.

Dans un mode de réalisation particulier de la présente invention, la séquence d'acide nucléique d'intérêt code pour un facteur de croissance des fibroblastes acide (aFGF). Le cDNA de la forme native du gène du aFGF humain a été identifié, séquencé et cloné (Jaye et al., 1986 et 1987). Ce cDNA peut coder pour différentes formes de aFGF, selon la présence de délétions dans la partie N-terminale, et notamment des formes comprenant 154, 140 ou même 134 acides aminés. Par ailleurs, des variants naturels ou artificiels peuvent également être produits, résultant de modifications par délétion, mutation et/ou addition d'une ou plusieurs paires de bases dans la séquence du gène natif (par exemple d'une méthionine N-terminale). Dans un mode de réalisation tout à fait spécifique de la présente invention, la séquence d'acide nucléique d'intérêt code pour le aFGF(154). A titre d'exemple spécifique, on peut citer le plasmide pXL2435 présentant la séquence SEQ ID n° 1.

Un autre objet de la présente invention concerne un procédé de production de protéines recombinantes. Plus particulièrement, le procédé de production selon l'invention consiste à cultiver une bactérie contenant :
- un plasmide tel que décrit ci-avant portant une séquence d'acide nucléique codant pour ladite protéine, et,
- le gène de l'ARN polymérase du bactériophage T7,
dans des conditions permettant l'expression de la séquence d'acide nucléique.

Comme indiqué ci-avant, l'intérêt du système réside notamment dans l'utilisation d'un promoteur du bactériophage T7 reconnu spécifiquement par l'ARN polymérase du même bactériophage T7. La bactérie utilisée doit donc comporter, en plus du plasmide selon l'invention, une cassette permettant l'expression de l'ARN polymérase du bactériophage T7. Cette cassette peut être soit intégrée au génome de la bactérie utilisée (souche E. coli BL21,DE3), soit portée par un second plasmide ou un phage, soit encore présente sur le plasmide de l'invention. Avantageusement, dans la cassette d'expression, le gène codant pour l'ARN polymérase du bactériophage T7 est placé sous le contrôle d'un promoteur inductible tel que le promoteur lac, trp ou recA. Ceci permet en effet d'induire de manière contrôlée la production d'ARN polymérase dans la cellule, et donc de contrôler l'expression de la séquence d'acide nucléique d'intérêt placée sous le contrôle du promoteur spécifique de ladite ARN polymérase. Préférentiellement, le promoteur inductible contrôlant l'expression de l'ARN polymérase du bactériophage T7 est le promoteur PlacUV5 d'E, coli, qui est induit spécifiquement en présence d'IPTG.

Comme indiqué plus en détail dans les exemples, le procédé de l'invention permet ainsi une production, à des taux élevés et de manière reproductible, de protéines non modifiées. Ce procédé permet ainsi une production industrielle de protéines recombinantes de qualité pharmaceutique.

Le procédé selon la présente invention est tout particulièrement adapté pour la production de facteurs de croissance des fibroblastes recombinants (aFGF, bFGF notamment). Ainsi, un objet particulier de la présente invention réside dans un procédé de préparation de aFGF recombinant selon lequel on cultive une bactérie contenant le plasmide pXL2435 et le gène de l'ARN polymérase du bactériophage T7 dans des conditions permettant l'expression de la séquence d'acide nucléique, et on récupère le aFGF produit.

La présente invention sera décrite plus complètement à l'aide des exemples qui suivent, qui doivent être considérés comme illustratifs et non limitatifs.

### Abréviations utilisées :

aFGF : facteur de croissance acide des fibroblastes
bp : paire de bases
DO : densité optique
E. coli : Escherichia coli
IPTG : isopropylthio-β-D-galactoside
kb : kilo bases
kDa : kilo daltons
Km : kanamycine
LB : Luria-Bertani medium
PAGE-SDS : électrophorèse en gel contenant de l'acrylamide, du N,N'-methylenebisacrylamide et du sodium dodécyl sulfate.
T7 : bactériophage T7

### Légendes des figures :

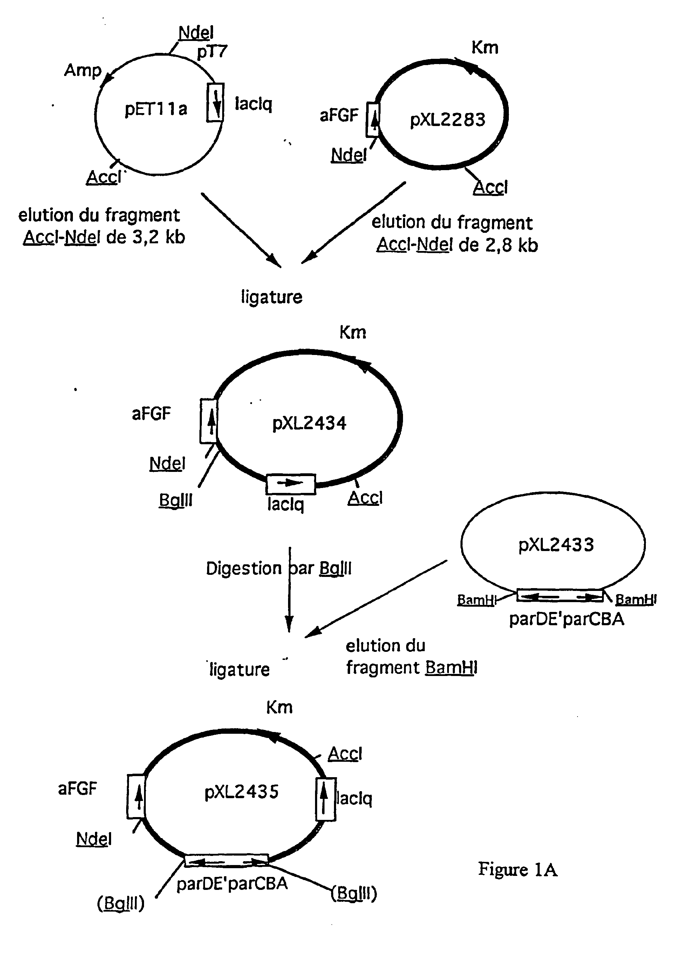

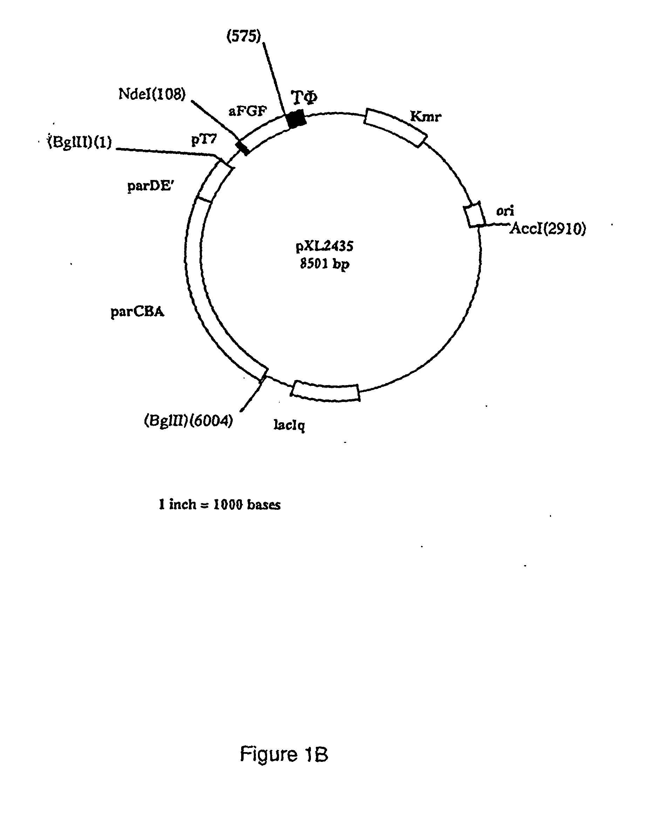

**Figure 1** **:** Construction du plasmide pXL2435.
   A- Schéma des constructions : Les fragments AccI-NdeI de 3,2 kb du pET11a et 2,8 kb du pXL2283 ont été ligaturés pour générer le plasmide pXL2434 ; le plasmide pXL2434 digéré par BglII a été ligaturé avec le fragment BamHI de 2,4 kb du pXL2433 pour créer le pXL2435.
   B- Carte du plasmide pXL2435 : *aFGF* : gène codant pour le aFGF ; Ampr : gène de résistance à l'ampicilline; Kmr : gène de résistance à la kanamycine ; *lacI^{q}* : gène codant pour une synthèse élevée du répresseur Lac ; ori : origine de réplication de ColE1 ; *parDE'parCBA* : gènes du locus par de RP4 ; pT7 : promoteur Φ10 de T7 et opérateur lacO ; TΦ : terminateur TΦ de T7. Les flèches indiquent la direction de transcription des gènes. Les sites des enzymes de restriction indiqués sont ceux qui ont été utilisés lors dès clonages. Les chiffres écrits entre parenthèse correspondent aux positions en paire de bases sur la séquence SEQ ID n°1.

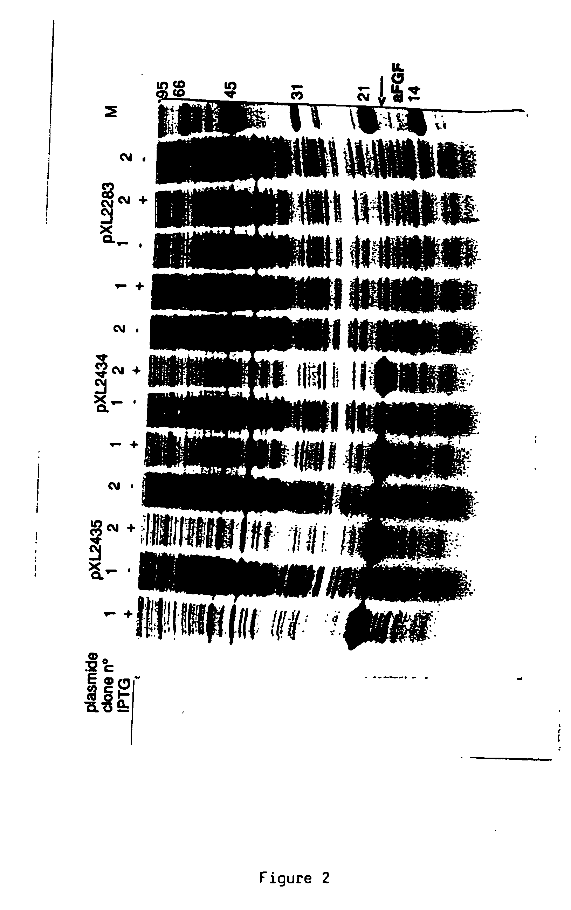

**Figure 2** **:** Visualisation de la protéine aFGF produite par E. coli BL21, DE3 en absence d'antibiotique et en présence du plasmide pXL2283 ou pXL2434 ou pXL2435 après induction avec de l'IPTG. Les extraits cellulaires totaux sont déposés sur le gel. M : marqueur de poids moléculaires qui sont indiqués en kDa, la flèche indique le poids moléculaire du aFGF.
   **Séquence SEQ ID n°1 :** Séquence nucléotidique du plasmide pXL2435 de 8501 bp. La position 1 sur la séquence correspond au site de coupure de BglII du pET11a. Le gène aFGF se situe entre les positions 108 à 575, le locus par entre les positions 6038 et 8499 c'est-à-dire parE' de 8248 à 8499, parD de 8001 à 8252, parC de 7850 à 7557, parB de 7560 à 7027 et parA de 7066 à 6407; le promoteur Φ10 de T7 entre les positions 20 et 36, lacO entre les positions 39 et 63, le terminateur TΦ de T7 entre les positions 586 et 708 et le gène lacI^{q} entre les positions 5636 et 4554.

### Techniques générales de clonage, de biologie moléculaire et de biochimie.

Les méthodes classiques de biologie moléculaire telles que la centrifugation d'ADN plasmidique en gradient de chlorure de césium-bromure d'éthidium, les digestions par des enzymes de restriction, l'électrophorèse sur gel, l'électroélution des fragments d'ADN à partir de gels d'agarose, la transformation dans E. coli, la précipitation des acides nucléiques etc, sont décrites dans la littérature (Sambrook et al., 1989). Les enzymes de restriction ont été fournies par New-England Biolabs (Biolabs), Bethesda Reseach Laboratories (BRL) ou Amersham Ltd (Amersham).

Pour les ligatures, les fragments d'ADN sont séparés selon leur taille sur des gels d'agarose à 0,7 %, purifiés par électroélution, extraits au phénol, précipités à l'éthanol puis incubés dans un tampon Tris-HCl pH 7.4 50 mM, MgCl₂ 10 mM, DTT 10 mM, ATP 2 mM, en présence d'ADN ligase du phage T4 (Biolabs). Les ADN ligaturés ou les ADN plasmidiques purs sont utilisés pour transformer la souche rendue compétente: E. coli BL21, DE3 [F- ompT hsdS_{B}(r_{B}⁻m_{B}⁻) gal dcm (DE3)] (Studier et al., 1990) et E. coli TG1 [Δ(lac proA,B), supE, thi, hsdD5/ F traD36, proA⁺, B⁺, lacI^{q}, lacZΔM15] (Gibson, 1984). Les ADN plasmidiques sont purifiés suivant la technique de la lyse alcaline décrite par Sambrook et al., 1989.

Le milieu de culture LB est utilisé pour la partie bactériologique (Sambrook et al., 1989). Les dilutions de la suspension bactérienne sont ensuite étalées sur des boites de mileu LB supplémentées avec, si nécessaire, de la kanamycine à 50 mg/l.

Le protocole décrit par Studier et al., 1990 est utilisé pour produire du aFGF à l'aide de la souche BL21, DE3 portant un plasmide d'expression du aFGF, (pXL2283 ou pXL2434 ou pXL2435 décrit dans l'exemple 1).

La production du aFGF est quantifiée sur des extraits cellulaires totaux. Après lyse des bactéries, les échantillons sont déposés sur un gel SDS-PAGE à 15% qui, après électrophorèse, est coloré au bleu de Coomassie (Denèfle et al., 1987). Avec les échantillons issus des souches produisant le aFGF une bande d'un poids moléculaire apparent de 16 kDa est observée. Un transfert semi-sec sur membrane de nitrocellulose est réalisé (Sambrook et al., 1989), la membrane est traitée selon le protocole du kit Vectastain (Biosys SA, France) pour permettre une détection du aFGF immunologique (anticorps anti-aFGF bovin obtenu chez le lapin est vendu par Sigma, France; et anti-IgG de lapin) colorimétrique à l'aide du complexe avidine péroxidase biotinylée. Un autre transfert semi-sec sur membrane Pro-Blott est réalisé (Matsudaira, 1987), la membrane est colorée au bleu de Coomassie et la bande de poids moléculaire apparent de 16 kDa est excisée. Cette partie de membrane est soumise à la dégradation d'Edman, 1956 à l'aide d'un microséquenceur d'Applied Biosystems modèle 477A possédant un analyseur 120/7 en ligne et utilisé selon les instructions du constructeur.

### Exemple 1- Construction d'un plasmide stable et régulé d'expression du cDNA du aFGF humain.

Cet exemple décrit les constructions réalisées pour obtenir un plasmide stable et très régulé permettant la production du aFGF chez E. coli.

Le cDNA de la forme native du gène du aFGF humain ( 470 pb) (Jaye et al., 1986 et 1987) a été identifié, séquencé et cloné dans le vecteur d'expression pET9 (Studier et al., 1990) aux sites NdeI et BamHI pour générer le plasmide d'expression pMJ42, nommé dans ce qui suit pXL2283 (voir figure 1). L'insert NdeI-AccI de 2,8 kb du plasmide pXL2283 a été ligaturé avec le fragment NdeI-AccI de 3,2 kb du plasmide pET11a (Studier et al., 1990) pour créer le plasmide d'expression pXL2434. Ce plasmide est différent du pXL2283 par la présence du gène lacI^{q} et de l'opérateur lacO, permettant d'obtenir une expression plus régulée du aFGF. Des sites BamHI ont ensuite été introduits aux extrémités du fragment parCBA-parDE' de 2,46 kb issu du plasmide pGMA28 (Gerlitz et al., 1990). Pour cela, le fragment SphI-SphI-BamHI de 2,46 kb du pGMA28 a été cloné dans le plasmide pUC18 (Yanish-Perron et al., 1985) digéré par SphI-BamHI pour générer le plasmide pGMA60 (H. Schwab et al., communication personnelle). Le fragment de 2,46 kb HindIII-EcoRI du plasmide pGMA60 a ensuite été cloné dans le plasmide pMTL22 (Chambers et al., 1988) digéré par HindIII et EcoRI pour générer pXL2433. L'insert BamHI (parCBA-parDE') du plasmide pXL2433 a été introduit au site BglII du plasmide pXL2434 pour créer le plasmide pXL2435. Sur la figure 1, un schéma des constructions et la carte du plasmide pXL2435 sont représentés et la séquence de 8501 pb du plasmide pXL2435 est décrite (SEQ ID n° 1). Alors que la séquence décrite dans l'article de Gerlitz et al., 1990 n'est que partielle (125 paires de bases sont à ajouter en 3'), cette partie 3' de la séquence a été vérifiée sur les plasmides pXL2433 et pXL2435 et sur la séquence SEQ ID n° 1. Un récapitulatif des caractéristiques des trois plasmides d'expression est présenté sur le tableau 1.

**Tableau 1. Caractéristiques des plasmides d'expression**

| **Plasmides** | | **pXL2283 ou pMJ42** | **pXL2434** | **pXL2435** |
|---|---|---|---|---|
| taille (kb) | | 4.8 | 6 | 8.5 |
| dérivé de | | pBR322 | pBR322 | pBR322 |
| résistance à la | | kanamycine | kanamycine | kanamycine |
| promoteur | pT7 | φ10 de T7 | φ10 de T7 | φ10 de T7 |
| | lacO | - | + | + |
| terminateur Tφ | | Tφ de T7 | Tφ de T7 | Tφ de T7 |
| cDNA du aFGF | | + | + | + |
| lacI ^{q} | | - | + | + |
| parCBA-parDE' | | - | - | + |

### Exemple 2- Stabilité du plasmide d'expression au cours de la production du aFGF

Cet exemple décrit la production, en milieu liquide, du aFGF à partir d'une souche de E. coli recombinante, possédant un plasmide d'expression du aFGF, T7-polymérase dépendant, et qui est cultivée en absence de sélection du plasmide. Après production du aFGF, la présence du plasmide est suivie par la résistance des bactéries à la kanamycine dont le gène de résistance est porté par les plasmides décrits dans l'exemple 1. Enfin la protéine aFGF produite est caractérisée par des méthodes de biochimie classiques.

### Exemple 2.1-Stabilité du plasmide d'expression en absence des conditions d'induction de la production de aFGF.

Les plasmides pXL2283, pXL2434 et pXL2435 sont introduits par transformation dans la souche BL21, DE3 (Studier et al., 1990). Parmi les transformants qui sont sélectionnés sur milieu gélosé LB contenant de la kanamycine, deux transformants, pris au hasard, sont cultivés 16 heures en milieu liquide LB en présence de kanamycine. La culture est diluée au 1/100ème et cultivée en milieu LB en présence de kanamycine jusqu'à ce que la densité optique à 600 nm soit comprise entre 0,2 à 0,6. Une dilution des cultures au 1/100ème en milieu LB est réalisée dans 10 ml de milieu, et les souches sont cultivées jusqu'à une DO comprise entre 0,17 à 0,6. Cette dilution-croissance des bactéries, dans le milieu sans antibiotique à 37°C, est répétée six fois et correspond, au total, à un nombre de générations de plus de 30, obtenu après deux jours. Les bactéries sont étalées sur milieu gélosé LB. Après croissance, 100 clones sont striés sur les milieux gélosés LB et LB contenant de la Km. Sur le tableau 2 figure la fréquence de clones résistants à la Km après au moins 30 générations sans pression de sélection du plasmide.

Cet exemple montre que le fragment par de RP4 stabilise le plasmide en absence de pression de sélection, même si cet effet de stabilisation n'est que modéré.

### Exemple 2.2-Stabilité du plasmide d'expression dans les conditions d'induction de la production de aFGF.

Les bactéries cultivées pendant au moins 30 générations en absence d'antibiotique (comme cela est décrit à l'exemple 2.1) sont diluées au 1/100ème en milieu LB, et cultivées 12 heures puis diluées alors au 1/100ème en milieu LB. Lorsque les bactéries sont poussées jusqu'à une DO de 0,5 à 0,8; 1mM d'IPTG sont ajoutés et les bactéries sont cultivées pendant 4 heures pour permettre la production du aFGF, qui est quantifiée sur les extraits cellulaires totaux déposés sur gel SDS-PAGE à 15 %, coloré au bleu de Coomassie (voir figure 2). Les bactéries sont étalées sur milieu gélosé LB. Après croissance, 100 clones sont striés sur les milieux gélosés LB et LB contenant de la kanamycine. Sur le tableau 2 figure la fréquence des clones résistants à la kanamycine après production du aFGF sans pression de sélection du plasmide.

Cet exemple montre sans ambiguïté que le fragment par induit une stabilité ségrégationnelle très importante suite à l'expression du aFGF en utilisant le système d'expression dépendant de la polymérase du phage T7.

**Tableau 2. Pourcentage des clones résistants à la kanamycine après croissance seulement ou croissance (au moins 30 générations) puis expression, sans pression de sélection du plasmide**

| Culture dilution sans Kanamycine | | | Culture expression sans Kanamycine | | |
|---|---|---|---|---|---|
| plasmide pXL | nombre de générations | % clones résistants à la Km | nombre de générations | % clones résistants à la Km | Production de aFGF |
| 2283 (n°1) | 30 | 20 | 45 | 0,1 | ε |
| 2283 (n°2) | 30 | 10 | 45 | 0,2 | ε |
| 2434 (n°1) | 40 | 100 | 45 | 1 | ++ |
| 2434 (n°2) | 40 | 99 | 45 | 5 | ++ |
| 2435 (n°1) | 40 | 100 | 45 | 99 | ++ |
| 2435 (n°1) | 40 | 100 | 45 | 100 | ++ |

| | | | | | |
|---|---|---|---|---|---|
| ε : expression du aFGF non détectée après coloration au bleu de Coomassie ++ : expression très élevée du aFGF environ 10 % des protéines totales. | | | | | |

### Exemple 2.3-Caractérisation de la protéine aFGF produite en présence du plasmide d'expression du aFGF.

Dans les conditions d'induction de production décrites dans l'exemple 2.2, la protéine aFGF obtenue est visualisée, voir figure 2, sur gel PAGE-SDS à 15 % colorée au bleu de Coomassie, et migre avec un poids moléculaire apparent de 16 kDa, en accord avec les données biochimiques décrites et la séquence publiée (Jaye et al., 1986 et 1987). De plus cette protéine est révélée par détection immunologique colorimétrique utilisant des anticorps anti-aFGF bovin. La séquence N-terminale de la protéine produite avec la souche BL21, DE3 pXL2435 a ensuite été déterminée à partir de l'extrait total qui a été purifié par électrophorèse PAGE-SDS, comme cela a été décrit dans les techniques générales de biochimie. La séquence obtenue est A-E-G-E-I-T-T-F-T-A-L-T (SEQ ID N°2), elle est identique à la séquence N-terminale de la protéine native (Jaye et al., 1986) et la méthionine terminale a été coupée par la méthionylaminopeptidase de E. coli comme cela a été discuté par Hirel et al., 1989.

Cet exemple montre donc que la protéine produite à l'aide d'un plasmide d'expression stable chez E. coli décrit dans l'exemple 2.2, est la protéine aFGF, et que sa séquence N-terminale n'est pas tronquée.

### LISTE DE SEQUENCES

(1) INFORMATION GENERALE:
   (i) DEPOSANT:
      (A) NOM: RHONE-POULENC RORER S.A.
      (B) RUE: 20, avenue R. ARON
      (C) VILLE: ANTONY
      (E) PAYS: FRANCE
      (F) CODE POSTAL: 920165
   (ii) TITRE DE L' INVENTION: Procédé de production de protéines recombinantes, plasmides et cellules modifiées.
   (iii) NOMBRE DE SEQUENCES: 2
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 8501 paires de bases
      (B) TYPE: acide nucléique
      (C) NOMBRE DE BRINS: double
      (D) CONFIGURATION: circulaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 1:
   GATCTCGATC CCGCGAAATT AATACGACTC ACTATAGGGG AATTGTGAGC GGATAACAAT      60
   TCCCCTCTAG AAATAATTTT GTTTAACTTT AAGAAGGAGA TATACATATG GCTGAAGGGG     120
   AAATCACCAC CTTCACAGCC CTGACCGAGA AGTTTAATCT GCCTCCAGGG AATTACAAGA     180
   AGCCCAAACT CCTCTACTGT AGCAACGGGG GCCACTTCCT GAGGATCCTT CCGGATGGCA     240
   CAGTGGATGG GACAAGGGAC AGGAGCGACC AGCACATTCA GCTGCAGCTC AGTGCGGAAA     300
   GCGTGGGGGA GGTGTATATA AAGAGTACCG AGACTGGCCA GTACTTGGCC ATGGACACCG     360
   ACGGGCTTTT ATACGGCTCA CAGACACCAA ATGAGGAATG TTTGTTCCTG GAAAGGCTGG     420
   AGGAGAACCA TTACAACACC TATATATCCA AGAAGCATGC AGAGAAGAAT TGGTTTGTTG     480
   GCCTCAAGAA GAATGGGAGC TGCAAACGCG GTCCTCGGAC TCACTATGGC CAGAAAGCAA     540
   TCTTGTTTCT CCCCCTGCCA GTCTCTTCTG ATTAAAGAGA TCCGGCTGCT AACAAAGCCC     600
   GAAAGGAAGC TGAGTTGGCT GCTGCCACCG CTGAGCAATA ACTAGCATAA CCCCTTGGGG     660
   CCTCTAAACG GGTCTTGAGG GGTTTTTTGC TGAAAGGAGG AACTATATCC GGATATCCAC     720
   AGGACGGGTG TGGTCGCCAT GATCGCGTAG TCGATAGTGG CTCCAAGTAG CGAAGCGAGC 780
   AGGACTGGGC GGCGGCCAAA GCGGTCGGAC AGTGCTCCGA GAACGGGTGC GCATAGAAAT 840
   TGCATCAACG CATATAGCGC TAGCAGCACG CCATAGTGAC TGGCGATGCT GTCGGAATGG 900
   ACGATATCCC GCAAGAGGCC CGGCAGTACC GGCATAACCA AGCCTATGCC TACAGCATCC 960
   AGGGTGACGG TGCCGAGGAT GACGATGAGC GCATTGTTAG ATTTCATACA CGGTGCCTGA 1020
   CTGCGTTAGC AATTTAACTG TGATAAACTA CCGCATTAAA GCTTATCGAT GATAAGCTGT 1080
   CAAACATGAG AATTCTTAGA AAAACTCATC GAGCATCAAA TGAAACTGCA ATTTATTCAT 1140
   ATCAGGATTA TCAATACCAT ATTTTTGAAA AAGCCGTTTC TGTAATGAAG GAGAAAACTC 1200
   ACCGAGGCAG TTCCATAGGA TGGCAAGATC CTGGTATCGG TCTGCGATTC CGACTCGTCC 1260
   AACATCAATA CAACCTATTA ATTTCCCCTC GTCAAAAATA AGGTTATCAA GTGAGAAATC 1320
   ACCATGAGTG ACGACTGAAT CCGGTGAGAA TGGCAAAAGC TTATGCATTT CTTTCCAGAC 1380
   TTGTTCAACA GGCCAGCCAT TACGCTCGTC ATCAAAATCA CTCGCATCAA CCAAACCGTT 1440
   ATTCATTCGT GATTGCGCCT GAGCGAGACG AAATACGCGA TCGCTGTTAA AAGGACAATT 1500
   ACAAACAGGA ATCGAATGCA ACCGGCGCAG GAACACTGCC AGCGCATCAA CAATATTTTC 1560
   ACCTGAATCA GGATATTCTT CTAATACCTG GAATGCTGTT TTCCCGGGGA TCGCAGTGGT 1620
   GAGTAACCAT GCATCATCAG GAGTACGGAT AAAATGCTTG ATGGTCGGAA GAGGCATAAA 1680
   TTCCGTCAGC CAGTTTAGTC TGACCATCTC ATCTGTAACA TCATTGGCAA CGCTACCTTT 1740
   GCCATGTTTC AGAAACAACT CTGGCGCATC GGGCTTCCCA TACAATCGAT AGATTGTCGC 1800
   ACCTGATTGC CCGACATTAT CGCGAGCCCA TTTATACCCA TATAAATCAG CATCCATGTT 1860
   GGAATTTAAT CGCGGCCTCG AGCAAGACGT TTCCCGTTGA ATATGGCTCA TAACACCCCT 1920
   TGTATTACTG TTTATGTAAG CAGACAGTTT TATTGTTCAT GACCAAAATC CCTTAACGTG 1980
   AGTTTTCGTT CCACTGAGCG TCAGACCCCG TAGAAAAGAT CAAAGGATCT TCTTGAGATC 2040
   CTTTTTTTCT GCGCGTAATC TGCTGCTTGC AAACAAAAAA ACCACCGCTA CCAGCGGTGG 2100
   TTTGTTTGCC GGATCAAGAG CTACCAACTC TTTTTCCGAA GGTAACTGGC TTCAGCAGAG 2160
   CGCAGATACC AAATACTGTC CTTCTAGTGT AGCCGTAGTT AGGCCACCAC TTCAAGAACT 2220
   CTGTAGCACC GCCTACATAC CTCGCTCTGC TAATCCTGTT ACCAGTGGCT GCTGCCAGTG 2280
   GCGATAAGTC GTGTCTTACC GGGTTGGACT CAAGACGATA GTTACCGGAT AAGGCGCAGC 2340
   GGTCGGGCTG AACGGGGGGT TCGTGCACAC AGCCCAGCTT GGAGCGAACG ACCTACACCG 2400
   AACTGAGATA CCTACAGCGT GAGCTATGAG AAAGCGCCAC GCTTCCCGAA GGGAGAAAGG 2460
   CGGACAGGTA TCCGGTAAGC GGCAGGGTCG GAACAGGAGA GCGCACGAGG GAGCTTCCAG 2520
   GGGGAAACGC CTGGTATCTT TATAGTCCTG TCGGGTTTCG CCACCTCTGA CTTGAGCGTC 2580
   GATTTTTGTG ATGCTCGTCA GGGGGGCGGA GCCTATGGAA AAACGCCAGC AACGCGGCCT 2640
   TTTTACGGTT CCTGGCCTTT TGCTGGCCTT TTGCTCACAT GTTCTTTCCT GCGTTATCCC 2700
   CTGATTCTGT GGATAACCGT ATTACCGCCT TTGAGTGAGC TGATACCGCT CGCCGCAGCC 2760
   GAACGACCGA GCGCAGCGAG TCAGTGAGCG AGGAAGCGGA AGAGCGCCTG ATGCGGTATT 2820
   TTCTCCTTAC GCATCTGTGC GGTATTTCAC ACCGCATATA TGGTGCACTC TCAGTACAAT 2880
   CTGCTCTGAT GCCGCATAGT TAAGCCAGTA TACACTCCGC TATCGCTACG TGACTGGGTC 2940
   ATGGCTGCGC CCCGACACCC GCCAACACCC GCTGACGCGC CCTGACGGGC TTGTCTGCTC 3000
   CCGGCATCCG CTTACAGACA AGCTGTGACC GTCTCCGGGA GCTGCATGTG TCAGAGGTTT 3060
   TCACCGTCAT CACCGAAACG CGCGAGGCAG CTGCGGTAAA GCTCATCAGC GTGGTCGTGA 3120
   AGCGATTCAC AGATGTCTGC CTGTTCATCC GCGTCCAGCT CGTTGAGTTT CTCCAGAAGC 3180
   GTTAATGTCT GGCTTCTGAT AAAGCGGGCC ATGTTAAGGG CGGTTTTTTC CTGTTTGGTC 3240
   ACTGATGCCT CCGTGTAAGG GGGATTTCTG TTCATGGGGG TAATGATACC GATGAAACGA 3300
   GAGAGGATGC TCACGATACG GGTTACTGAT GATGAACATG CCCGGTTACT GGAACGTTGT 3360
   GAGGGTAAAC AACTGGCGGT ATGGATGCGG CGGGACCAGA GAAAAATCAC TCAGGGTCAA 3420
   TGCCAGCGCT TCGTTAATAC AGATGTAGGT GTTCCACAGG GTAGCCAGCA GCATCCTGCG 3480
   ATGCAGATCC GGAACATAAT GGTGCAGGGC GCTGACTTCC GCGTTTCCAG ACTTTACGAA 3540
   ACACGGAAAC CGAAGACCAT TCATGTTGTT GCTCAGGTCG CAGACGTTTT GCAGCAGCAG 3600
   TCGCTTCACG TTCGCTCGCG TATCGGTGAT TCATTCTGCT AACCAGTAAG GCAACCCCGC 3660
   CAGCCTAGCC GGGTCCTCAA CGACAGGAGC ACGATCATGC GCACCCGTGG CCAGGACCCA 3720
   ACGCTGCCCG AGATGCGCCG CGTGCGGCTG CTGGAGATGG CGGACGCGAT GGATATGTTC 3780
   TGCCAAGGGT TGGTTTGCGC ATTCACAGTT CTCCGCAAGA ATTGATTGGC TCCAATTCTT 3840
   GGAGTGGTGA ATCCGTTAGC GAGGTGCCGC CGGCTTCCAT TCAGGTCGAG GTGGCCCGGC 3900
   TCCATGCACC GCGACGCAAC GCGGGGAGGC AGACAAGGTA TAGGGCGGCG CCTACAATCC 3960
   ATGCCAACCC GTTCCATGTG CTCGCCGAGG CGGCATAAAT CGCCGTGACG ATCAGCGGTC 4020
   CAGTGATCGA AGTTAGGCTG GTAAGAGCCG CGAGCGATCC TTGAAGCTGT CCCTGATGGT 4080
   CGTCATCTAC CTGCCTGGAC AGCATGGCCT GCAACGCGGG CATCCCGATG CCGCCGGAAG 4140
   CGAGAAGAAT CATAATGGGG AAGGCCATCC AGCCTCGCGT CGCGAACGCC AGCAAGACGT 4200
   AGCCCAGCGC GTCGGCCGCC ATGCCGGCGA TAATGGCCTG CTTCTCGCCG AAACGTTTGG 4260
   TGGCGGGACC AGTGACGAAG GCTTGAGCGA GGGCGTGCAA GATTCCGAAT ACCGCAAGCG 4320
   ACAGGCCGAT CATCGTCGCG CTCCAGCGAA AGCGGTCCTC GCCGAAAATG ACCCAGAGCG 4380
   CTGCCGGCAC CTGTCCTACG AGTTGCATGA TAAAGAAGAC AGTCATAAGT GCGGCGACGA 4440
   TAGTCATGCC CCGCGCCCAC CGGAAGGAGC TGACTGGGTT GAAGGCTCTC AAGGGCATCG 4500
   GTCGAGATCC CGGTGCCTAA TGAGTGAGCT AACTTACATT AATTGCGTTG CGCTCACTGC 4560
   CCGCTTTCCA GTCGGGAAAC CTGTCGTGCC AGCTGCATTA ATGAATCGGC CAACGCGCGG 4620
   GGAGAGGCGG TTTGCGTATT GGGCGCCAGG GTGGTTTTTC TTTTCACCAG TGAGACGGGC 4680
   AACAGCTGAT TGCCCTTCAC CGCCTGGCCC TGAGAGAGTT GCAGCAAGCG GTCCACGCTG 4740
   GTTTGCCCCA GCAGGCGAAA ATCCTGTTTG ATGGTGGTTA ACGGCGGGAT ATAACATGAG 4800
   CTGTCTTCGG TATCGTCGTA TCCCACTACC GAGATATCCG CACCAACGCG CAGCCCGGAC 4860
   TCGGTAATGG CGCGCATTGC GCCCAGCGCC ATCTGATCGT TGGCAACCAG CATCGCAGTG 4920
   GGAACGATGC CCTCATTCAG CATTTGCATG GTTTGTTGAA AACCGGACAT GGCACTCCAG 4980
   TCGCCTTCCC GTTCCGCTAT CGGCTGAATT TGATTGCGAG TGAGATATTT ATGCCAGCCA 5040
   GCCAGACGCA GACGCGCCGA GACAGAACTT AATGGGCCCG CTAACAGCGC GATTTGCTGG 5100
   TGACCCAATG CGACCAGATG CTCCACGCCC AGTCGCGTAC CGTCTTCATG GGAGAAAATA 5160
   ATACTGTTGA TGGGTGTCTG GTCAGAGACA TCAAGAAATA ACGCCGGAAC ATTAGTGCAG 5220
   GCAGCTTCCA CAGCAATGGC ATCCTGGTCA TCCAGCGGAT AGTTAATGAT CAGCCCACTG 5280
   ACGCGTTGCG CGAGAAGATT GTGCACCGCC GCTTTACAGG CTTCGACGCC GCTTCGTTCT 5340
   ACCATCGACA CCACCACGCT GGCACCCAGT TGATCGGCGC GAGATTTAAT CGCCGCGACA 5400
   ATTTGCGACG GCGCGTGCAG GGCCAGACTG GAGGTGGCAA CGCCAATCAG CAACGACTGT 5460
   TTGCCCGCCA GTTGTTGTGC CACGCGGTTG GGAATGTAAT TCAGCTCCGC CATCGCCGCT 5520
   TCCACTTTTT CCCGCGTTTT CGCAGAAACG TGGCTGGCCT GGTTCACCAC GCGGGAAACG 5580
   GTCTGATAAG AGACACCGGC ATACTCTGCG ACATCGTATA ACGTTACTGG TTTCACATTC 5640
   ACCACCCTGA ATTGACTCTC TTCCGGGCGC TATCATGCCA TACCGCGAAA GGTTTTGCGC 5700
   CATTCGATGG TGTCCGGGAT CTCGACGCTC TCCCTTATGC GACTCCTGCA TTAGGAAGCA 5760
   GCCCAGTAGT AGGTTGAGGC CGTTGAGCAC CGCCGCCGCA AGGAATGGTG CATGCAAGGA 5820
   GATGGCGCCC AACAGTCCCC CGGCCACGGG GCCTGCCACC ATACCCACGC CGAAACAAGC 5880
   GCTCATGAGC CCGAAGTGGC GAGCCCGATC TTCCCCATCG GTGATGTCGG CGATATAGGC 5940
   GCCAGCAACC GCACCTGTGG CGCCGGTGAT GCCGGCCACG ATGCGTCCGG CGTAGAGGAT 6000
   CGAGATCCAT ATGACGTCGA CGCGTCTGCA GAAGCTTGCA TGCCAGCTTC TGGTTCGTCG 6060
   GCTGGGTGAT GGCGTCGGTT TTGGCCGGCG GCGTCGGCGC GATCGCCAGC GCGAAGCAAC 6120
   TGGCGTTCCT CGGCGAACAT AGCGGCATGG TGGCCTTCGG CTTCTTCCGC GACCAGGTGA 6180
   AGGACATGCA CTGCGATGCG GACGTGATCC TGGCCCGGTG GGATGAAAAG GCGAACTCGC 6240
   CGGTGGTCTA CCGCTGCCCG AAGGCGTACC TGCTCAACAG GTTCGCATCC GCGCCCTTCG 6300
   TGCCCTGGCC GGACTACACC GAGGGGGAAA GCGAGGATCT AGGTAGGGCG CTCGCAGCGG 6360
   CCCTGCGGGA CGCGAAAAGG TGAGAAAAGC CGGGCACTGC CCGGCTTTAT TTTTGCTGCT 6420
   GCGCGTTCCA GGCCGCCCAC ACTCGTTTGA CCTGGCTCGG GCTGCATCCG ACCAGCTTGG 6480
   CCGTCTTGGC AATGCTCGAT CCGCCGGAGC GAAGCGTGAT GATGCGGTCG TGCATGCCGG 6540
   CGTCACGTTT GCGGCCGGTG TAGCGGCCGG CGGCCTTCGC CAACTGGACA CCCTGACGTT 6600
   GACGCTCGCG CCGATCCTCG TAGTCGTCGC GGGCCATCTG CAAGGCGAGC TTCAAAAGCA 6660
   TGTCCTGGAC GGATTCCAGA ACGATTTTCG CCACTCCGTT CGCCTCGGCG GCCAGCTCCG 6720
   ACAGGTCCAC CACGCCAGGC ACGGCCAGCT TGGCCCCTTT GGCCCGGATC GACGCAACCA 6780
   GGCGCTCGGC CTCGGCCAAC GGCAAGCGGC TGATGCGGTC GATCTTCTCC GCAACGACGA 6840
   CTTCACCAGG TTGCAGGTCC GCGATCATGC GCAGCAGCTC GGGCCGGTCG GCGCGTGCGC 6900
   CGGACGCCTT CTCGCGGTAG ATGCCGGCGA CGTAGTACCC GGCGGCCCGC GTGGCCGCTA 6960
   CAAGGCTCTC CTGGCGTTCA AGATTCTGCT CGTCCGTACT GGCGCGCAGG TAGATGCGGG 7020
   CGACCTTCAA CCTTCGTCCC TCCGGTTGTT GCTCTCGCGT CGCCATTTCC ACGGCTCGAC 7080
   GGCGTGCGGA TCGGACCAGA GGCCGACGCG CTTGCCTCGC GCCTCCTGTT CGAGCCGCAG 7140
   CATTTCAGGG TCGGCCGCGC GGCCGTGGAA GCGATAGGCC CACGCCATGC CCTGGTGAAC 7200
   CATCGCGGCG TTGACGTTGC GCGGCTGCGG CGGCCGGCTG GCCAGCTCCA TGTTGACCCA 7260
   CACGGTGCCC AGCGTGCGGC CGTAACGGTC GGTGTCCTTC TCGTCGACCA GGACGTGCCG 7320
   GCGGAACACC ATGCCGGCCA GCGCCTGGCG CGCACGTTCG CCGAAGGCTT GCCGCTTTTC 7380
   CGGCGCGTCA ATGTCCACCA GGCGCACGCG CACCGGCTGC TTGTCTACCA GCACGTCGAT 7440
   GGTGTCGCCG TCGATGATGC GCACGACCTC GCCGCGCAGC TCGGCCCATG CCGGCGAGGC 7500
   AACGACCAGG ACGGCCAGCG CGGCAGCGGC GCGCAGCATG GCGTAGCTTC GGCGCTTCAT 7560
   GCGTGGCCCC ATTGCTGATG ATCGGGGTAC GCCAGGTGCA GCACTGCATC GAAATTGGCC 7620
   TTGCAGTAGC CGTCCAGCGC CACCCGCGAG CCGAACGCCG GCGAAAGGTA CTCGACCAGG 7680
   CCGGGCCGGT CGCGGACCTC GCGCCCCAGG ACGTGGATGC GCCGGCCGCG TGTGCCGTCG 7740
   GGTCCAGGCA CGAAGGCCAG CGCCTCGATG TTGAAGTCGA TGGATAGAAG TTGTCGGTAG 7800
   TGCTTGGCCG CCCTCATCGC GTCCCCCTTG GTCAAATTGG GTATACCCAT TTGGGCCTAG 7860
   TCTAGCCGGC ATGGCGCATT ACAGCAATAC GCAATTTAAA TGCGCCTAGC GCATTTTCCC 7920
   GACCTTAATG CGCCTCGCGC TGTAGCCTCA CGCCCACATA TGTGCTAATG TGGTTACGTG    7980
   TATTTTATGG AGGTTATCCA ATGAGCCGCC TGACAATCGA CATGACGGAC CAGCAGCACC    8040
   AGAGCCTGAA AGCCCTGGCC GCCTTGCAGG GCAAGACCAT TAAGCAATAC GCCCTCGAAC    8100
   GTCTGTTCCC CGGTGACGCT GATGCCGATC AGGCATGGCA GGAACTGAAA ACCATGCTGG    8160
   GGAACCGCAT CAACGATGGG CTTGCCGGCA AGGTGTCCAC CAAGAGCGTC GGCGAAATTC    8220
   TTGATGAAGA ACTCAGCGGG GATCGCGCTT GACGGCCTAC ATCCTCACGG CTGAGGCCGA    8280
   AGCCGATCTA CGCGGCATCA TCCGCTACAC GCGCCGGGAG TGGGGCGCGG CGCAGGTGCG    8340
   CCGCTATATC GCTAAGCTGG AACAGGGCAT AGCCAGGCTT GCCGCCGGCG AAGGCCCGTT    8400
   TAAGGACATG AGCGAACTCT TTCCCGCGCT GCGGATGGCC CGCTGCGAAC ACCACTACGT    8460
   TTTTTGCCTG CCGCGTGCGG GCGAACCCGC GTTGGTCGTC G                        8501
(2) INFORMATION POUR LA SEQ ID NO: 2:
   (i) CARACTERISTIQUES DE LA SEQUENCE:
      (A) LONGUEUR:12 acides aminés
      (B) TYPE: acide aminé
      (D)CONFIGURATION:linéaire
   (ii) TYPE DE MOLECULE: PROTEINE
   (iii) HYPOTHETIQUE: NON
   (xi) DESCRIPTION DE LA SEQUENCE: SEQ ID NO: 2:

### Références

Chambers, S., Prior, S., Barstow, D., and Minton, N. (1988) Gene 68: 139-149.
Denèfle P., Kovarik, S. , Guiton, J.D., Cartwright, T., and Mayaux, J.-F. (1987) Gene 56: 61-70.
Eberl L., Kristensen C., Givskov M., Grohmann E., Gerlitz M., and Schawb H. (1994) Mol. Microbiol. 12: 131-141.
Edman P. (1956) Acta Chemica Scandinavia 10: 761-768.
Gerlitz et al., (1990) J. Bact. 172: 6194-6203
Gibson T. (1984) Ph.D., University of Cambridge, Cambridge, England.
Hirel P., Schmitter P., Dessen P., and Blanquet S. (1989) Proc. Natl. Acad. Sci. 86: 8247-8251.
Jaye M., Howk R., Burgess W., Ricca G., Chui I., Ravera M., O'Brien S., Modi W., Maciag T., and Drohan W. (1986) Science 233: 541-545.
Jaye M., Burgess W., Shaw A., Drohan W. (1987) J. Biol. Chem. 262: 16612-16617.
Matsudaira P. (1987) J. Biol. Chem. 262: 10035-10038.
Roberts et al. (1994) J. Mol. Biol. 237: 35-51.
Sambrook J., Fritsch, E.F., and Maniatis, T. (1989) Molecular Cloning: a Laboratory Manual 2nd edn. Cold Spring Harbor, New York: Cold Spring Harbor Laboratory Press.
Studier, W. F., Rosenberg, A. H., Dunn, J. J., and Duberndorff, J. W. (1990) Methods Enzymo/. 185:89-60.
Yanisch-Perron, C., Vieira, J., Messing, J. (1985) Gene 33: 1033-119.

## Revendications

1. Plasmide d'expression **caractérisé en ce qu'**il comprend une séquence d'acide nucléique d'intérêt sous le contrôle d'un promoteur du bactériophage T7 et une région stabilisatrice comprenant tout ou partie de la région par du plasmide RP4 ou d'un dérivé de celle-ci et **en ce qu'**il comporte en outre l'opérateur lacO et le gène lacI^{q}.

2. Plasmide selon la revendication 1 **caractérisé en ce que** le promoteur du bactériophage T7 est le promoteur du gène 10.

3. Plasmide selon la revendication 1 **caractérisé en ce que** la région stabilisatrice comprend une partie de la région par du plasmide RP4.

4. Plasmide selon la revendication 3 **caractérisé en ce que** la région stabilisatrice est constituée du fragment compris entre les résidus 6038 et 8499 de la séquence SEQ ID n° 1.

5. Plasmide selon la revendication 1 **caractérisé en ce que** l'opérateur lacO est situé en aval du promoteur du bactériophage T7 et en amont de la séquence d'acide nucléique d'intérêt.

6. Plasmide selon l'une des revendications précédentes **caractérisé en ce que** la séquence d'acide nucléique d'intérêt code pour une protéine d'intérêt pharmaceutique, agroalimentaire ou utilisable en biocatalyse.

7. Plasmide selon la revendication 6 **caractérisé en ce que** la séquence d'acide nucléique d'intérêt code pour une protéine choisie parmi les enzymes, les dérivés sanguins, les hormones, les lymphokines, les facteurs de croissance, les neurotransmetteurs ou leurs précurseurs ou enzymes de synthèse, les facteurs trophiques, les apolipoprotéines, la dystrophine ou une minidystrophine, la protéine CFTR, les gènes suppresseurs de tumeurs les gènes codant pour des facteurs impliqués dans la coagulation ou les gènes intervenant dans la réparation de l'ADN.

8. Plasmide selon la revendication 7 **caractérisé en ce que** la séquence d'acide nucléique d'intérêt code pour on facteur de croissance acide des fibroblastes (aFGF).

9. Plasmide selon la revendication 8 **caractérisé en ce que** la séquence d'acide nucléique d'intérêt code pour le aFGF(154).

10. Plasmide pXL2435 présentant la séquence SEQ ID n° 1.

11. Procédé de production de protéine recombinant **caractérisé en ce que** l'on cultive une bactérie contenant :
- un plasmide selon l'une des revendications 1 à 10 portant une séquence d'acide nucléique codant pour ladite protéine, et,
- le gène de l'ARN polymérase du bactériophage T7.
dans des conditions permettant l'expression de la séquence d'acide nucléique.

12. Procédé selon la revendication 11 **caractérisé en ce que** le gène de l'ARN polymérase du bactériophage T7 est placé sous le contrôle d'un promoteur inductible.

13. Procédé selon les revendications 11 ou 12 **caractérisé en ce que** le gène de l'ARN polymérase du bactériophage T7 est intégré au génome de la bactérie utilisée.

14. Procédé selon l'une des revendications 11 à 13 **caractérisé en ce que** la bactérie est choisie parmi les souches d'E. coli.

15. Procédé selon la revendication 14 **caractérisé en ce que** la bactérie est la souche E. coli BL21, DE3.

16. Procédé selon les revendications 11 à 15 pour la production de aFGF recombinant.

17. Procédé de préparation de aFGF recombinant **caractérisé en ce que** l'on cultive une bactérie contenant le plasmide pXL2435 et le gène de l'ARN polymérase du bactériophage T7 dans des conditions permettant l'expression de la séquence d'acide nucléique, et on récupère le aFGF produit.

18. Bactérie transformée par un plasmide selon l'une des revendications 1 à 10.

## Claims

1. Expression plasmid, **characterized in that** it comprises a nucleic acid sequence of interest under the control of a T7 bacteriophage promoter and a stabilizing region comprising all or part of the par region of the RP4 plasmid or of a derivative thereof, and **in that** it also comprises the lacQ operator and the lacI^{q} gene.

2. Plasmid according to Claim 1, **characterized in that** the T7 bacteriophage promoter is the gene 10 promoter.

3. Plasmid according to Claim 1, **characterized in that** the stabilizing region comprises a part of the par region of the RP4 plasmid.

4. Plasmid according to Claim 3, **characterized in that** the stabilizing region comprises the fragment between residues 6038 and 8499 of the sequence SEQ ID No. 1.

5. Plasmid according to Claim 1, **characterized in that** the lacQ operator is located downstream of the T7 bacteriophage promoter and upstream of the nucleic acid sequence of interest.

6. Plasmid according to one of the preceding claims, **characterized in that** the nucleic acid sequence of interest encodes a protein which is of pharmaceutical or agrofoods interest or which can be used in biocatalysis.

7. Plasmid according to Claim 6, **characterized in that** the nucleic acid sequence of interest encodes a protein selected from enzymes, blood derivatives, hormones, lymphokines, growth factors, neurotransmitters or precursors of said neurotransmitters or enzymes for the synthesis of said neurotransmitters, trophic factors, apolipoproteins, dystrophin or minidystrophin, the CFTR protein, tumour suppressor genes, genes encoding factors involved in clotting or genes involved in DNA repair.

8. Plasmid according to Claim 7, **characterized in that** the nucleic acid sequence of interest encodes an acidic fibroblast growth factor (aFGF).

9. Plasmid according to Claim 8, **characterized in that** the nucleic acid sequence of interest encodes aFGF(154).

10. Plasmid pXL2435 having the sequence SEQ ID No. 1.

11. Method for producing a recombinant protein, **characterized in that** a bacterium containing:
- a plasmid according to one of Claims 1 to 10 carrying a nucleic acid sequence encoding said protein, and
- the RNA polymerase gene of the T7 bacteriophage, is cultured under conditions which allow the expression of the nucleic acid sequence.

12. Method according to Claim 11, **characterized in that** the RNA polymerase gene of the T7 bacteriophage is placed under the control of an inducible promoter.

13. Method according to Claim 11 or 12, **characterized in that** the RNA polymerase gene of the T7 bacteriophage is integrated into the genome of the bacterium used.

14. Method according to one of Claims 11 to 13, **characterized in that** the bacterium is selected from strains of E. coli.

15. Method according to Claim 14, **characterized in that** the bacterium is the E. coli BL21, DE3 strain.

16. Method according to Claims 11 to 15, for producing recombinant aFGF.

17. Method for preparing recombinant aFGF, **characterized in that** a bacterium containing the pXL2435 plasmid and the RNA polymerase gene of the T7 bacteriophage is cultured under conditions which allow the expression of the nucleic acid sequence, and the aFGF produced is recovered.

18. Bacterium transformed with a plasmid according to one of Claims 1 to 10.

## Patentansprüche

1. Expressionsplasmid, **dadurch gekennzeichnet, dass** es eine Nukleinsäuresequenz von Interesse unter der Kontrolle eines Bakteriophage-T7-Promotors und eine stabilisierende Region umfasst, welche die gesamte oder einen Teil der par-Region des Plasmids RP4 oder ein Derivat davon umfasst, und **dadurch**, dass es außerdem den lacO-Operator und das lacI^{q}-Gen umfasst.

2. Plasmid nach Anspruch 1, **dadurch gekennzeichnet, dass** der Bakteriophage-T7-Promotor der Promotor des Gens 10 ist.

3. Plasmid nach Anspruch 1, **dadurch gekennzeichnet, dass** die stabilisierende Region einen Teil der par-Region des Plasmids RP4 umfasst.

4. Plasmid nach Anspruch 3, **dadurch gekennzeichnet, dass** die stabilisierende Region aus dem Fragment zwischen den Resten 6038 und 8499 der Sequenz SEQ ID NR: 1 besteht.

5. Plasmid nach Anspruch 1, **dadurch gekennzeichnet, dass** der lacO-Operator sich stromabwärts des Bakteriophage-T7-Promoters und stromaufwärts der interessierenden Nukleinsäuresequenz befindet.

6. Plasmid nach einem der vorhergehenden Ansprüche, **dadurch gekennzeichnet, dass** die interessierende Nukleinsäuresequenz für ein Protein codiert, das für die Pharmazie, die Nahrungsmittelindustrie interessant oder zur Biokatalyse einsetzbar ist.

7. Plasmid nach Anspruch 6, **dadurch gekennzeichnet, dass** die interessierende Nukleinsäuresequenz für ein Protein codiert, das ausgewählt ist aus Enzymen, Blutderivaten, Hormonen, Lymphokinen, Wachstumsfaktoren, Neutrotransmittern oder ihren Vorläufern oder Syntheseenzymen, trophischen Faktoren, Apolipoproteinen, Dystrophin oder einem Minidystrophin, dem CFTR-Protein, Tumorsuppressorgenen, Genen, die für Faktoren codieren, die an der Blutgerinnung beteiligt sind, oder Genen, die an der DNA-Reparatur beteiligt sind.

8. Plasmid nach Anspruch 7, **dadurch gekennzeichnet, dass** die interessierende Nukleinsäuresequenz für einen sauren Fibroblasten-Wachstumsfaktor (aFGF) codiert.

9. Plasmid nach Anspruch 8, **dadurch gekennzeichnet, dass** die interessierende Nukleinsäuresequenz für aFGF(154) codiert.

10. Plasmid pXL2435 mit der Sequenz SEQ ID NR: 1.

11. Verfahren zur Produktion von rekombinantem Protein, **dadurch gekennzeichnet, dass** man ein Bakterium züchtet, das Folgendes enthält:
- ein Plasmid nach einem der Ansprüche 1 bis 10, das eine Nukleinsäuresequenz trägt, die für das Protein codiert, und
- das RNA-Polymerase-Gen des Bakteriophagen T7 unter Bedingungen züchtet, welche die Expression der Nukleinsäuresequenz gestatten.

12. Verfahren nach Anspruch 11, **dadurch gekennzeichnet, dass** das RNA-Polymerase-Gen des Bakteriophagen T7 unter die Kontrolle eines induzierbaren Promotors gestellt wird.

13. Verfahren nach Anspruch 11 oder 12, **dadurch gekennzeichnet, dass** das RNA-Polymerase-Gen des Bakteriophagen T7 in das Genom des verwendeten Bakteriums integriert ist.

14. Verfahren nach einem der Ansprüche 11 bis 13, **dadurch gekennzeichnet, dass** das Bakterium aus E.-coli-Stämmen ausgewählt ist.

15. Verfahren nach Anspruch 14, **dadurch gekennzeichnet, dass** das Bakterium der Stamm E. coli BL21, DE3 ist.

16. Verfahren nach den Ansprüchen 11 bis 15 zur Produktion von rekombinantem aFGF.

17. Verfahren zur Herstellung von rekombinantem aFGF, **dadurch gekennzeichnet, dass** man ein Bakterium, welches das Plasmid pXL2435 und das RNA-Polymerase-Gen des Bakteriophagen T7 enthält, unter Bedingungen züchtet, welche die Expression der Nukleinsäuresequenz gestatten, und den produzierten aFGF gewinnt.

18. Bakterium, das mit einem Plasmid nach einem der Ansprüche 1 bis 10 transformiert ist.
